# EUROPEAN PATENT SPECIFICATION

(11) **EP 1 221 321 B1**
(45) Date of publication and mention of the grant of the patent: **16.04.2008**
(21) Application number: 01130847.5
(22) Date of filing: 27.12.2001
(51) Int. Cl.: A61M 25/00

(54) **Method for making balloon catheter**
Verfahren zur Herstellung von Ballonkathetern
Procédé pour fabriquer des cathéters à ballonnet

(30) Priority: 03.01.2001 KR 2001000127; 23.06.2001 KR 2001036004
(43) Date of publication of application: 10.07.2002
(73) Proprietor: Lee, Keun-Ho, Buchoeon City, Kyungki-do (KR)
(72) Inventor: Lee, Keun-Ho, Buchoeon City, Kyungki-do (KR)
(74) Representative: Gervasi, Gemma

(56) References cited:
- FR-A- 2 673 110
- US-A- 3 452 756
- US-A- 3 832 253
- US-A- 3 926 705
- US-A- 5 137 671
- US-A- 5 795 332

## Description

### BACKGROUND OF THE INVENTION

### 1. Field of the Invention

The present invention relates to a method for making a silicon balloon catheter, and more particularly, to a method for making a silicon balloon catheter in which a first tube having its outer diameter slightly smaller than that of a desired catheter is formed by an extruding method, mold lubricant is coated at a portion of a balloon injection opening, a thin film type second tube is formed at the coated outer surface of the first tube by a second extruding, and then the catheter is vulcanized and cut. As a result, when liquid is injected to an expansion tube, the second tube is separated from the first tube, thereby performing a function as a balloon.

### 2. Description of the Related Art

In general, a catheter made of silicon is a thin and long tube being inserted into the human body in order to draw blood or inject medicine. For example, it is used a urine path tube, that is, it is inserted to bladder through urethra in order to make urine filled within the bladder discharge.

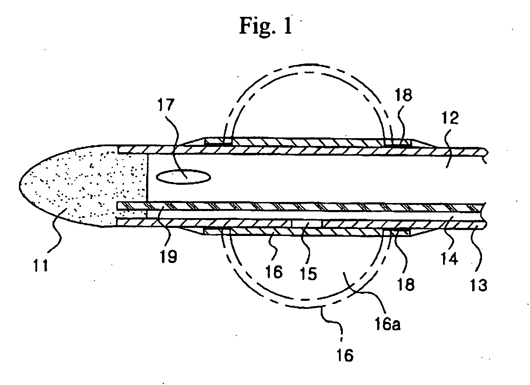

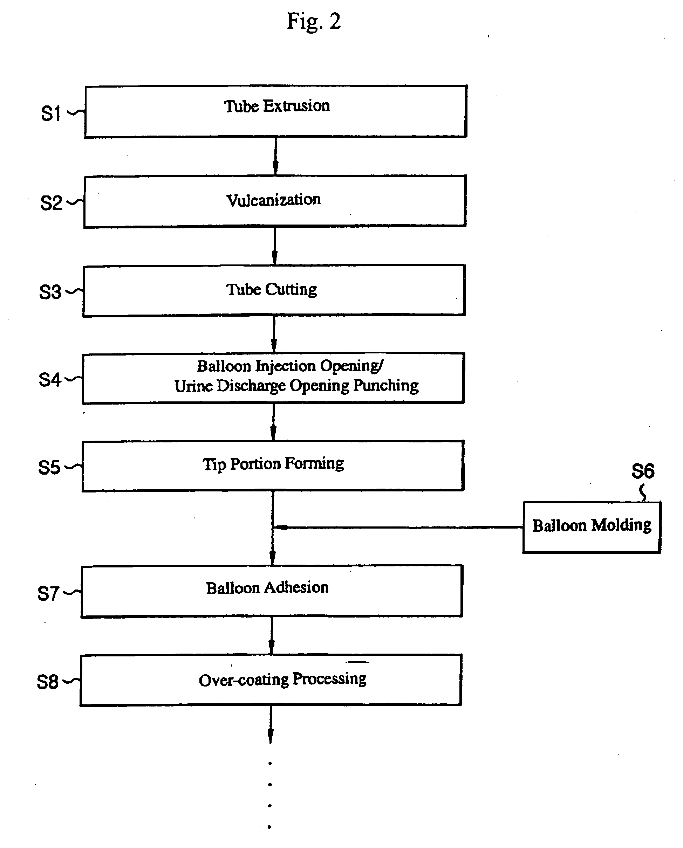

Figure 1 is a cross-sectional view illustrating a balloon catheter in accordance with a conventional art. Figure 2 is a block diagram illustrating a manufacturing method of a balloon catheter in accordance with a conventional art.

In a construction of the conventional art (referring to Figure 1), a tube 13 has a partition 19 between a discharge tube path 12 for discharging urine through a urine discharge opening 17 and an expansion tube 14 for expanding a balloon. A balloon layer 16 is adhered to an outside of the tube 13 by an adhesive 18. Also, the conventional catheter has a balloon injection opening 15 for making the expansion tube 14 and the inside 16a of the balloon communicate.

In order to manufacture the catheter as constructed above, firstly, the tube 13 is extruded in order to provide the discharge tube path 12 and an expansion tube 14 in step S1, thereafter vulcanized in step S2 and cut with a predetermined length in step S3.

Next, the balloon injection opening 15 and the urine discharge opening 17 are punched in step S4 and then a tip portion 11 is formed in step S5. Thereafter, the balloon 16 molded in a separate process, i.e., the step S6, is adhered by adhesive in step S7 and then over-coated in step S8.

However, in the catheter as described above, there are several disadvantages that since it is manufactured by an adhesion of a balloon molded by a separate process, the diameter of the balloon portion become thicker relatively in comparison with an another portion of the catheter. As a result, the portion caused a patient to feel a very big pain in the surgical operation and also sometimes there is a serious situation that the adhesive portion is apt to be separated. In FR 2 673 110 the ballon is formed by a second extrusion step followed by vulcanisation. The method comprises: extruding a first tube, punching injection openings, coating an anti-adhesive, extruding the ballon layer onto the first tube and subsequent vulcanisation; all steps are carried out in a continuous process, without cutting the first tube into sections before further processing.

Also, U.S. 5,137,671 discloses another catheter method, in which a tube 100 (fig.3a) includes a first tube 120 and a second tube 140.

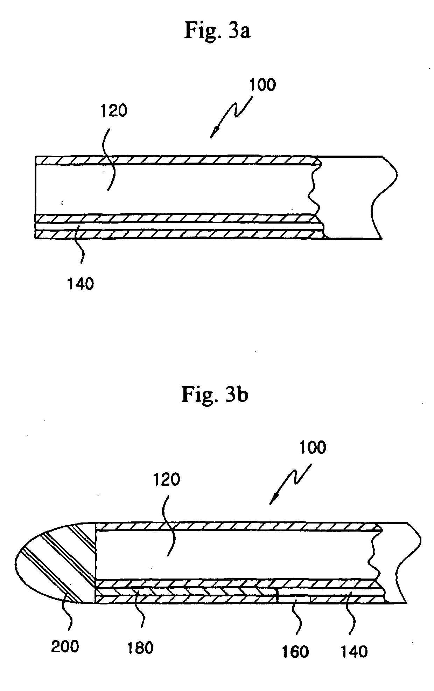

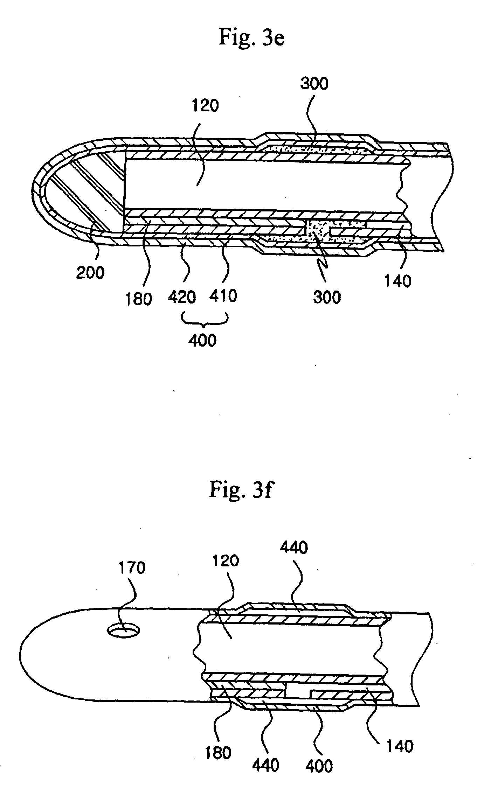

When the tube 100 as described above is provided, as shown in Figure 3b, a first opening 160 communicating the second tube 140 at the outer surface of the balloon expansion unit is punched. Thereafter, a polymerization type filling material 180 like silicon rubber is filled within the second tube 140 from an end (a lower end) of the tube 100 to the first opening 160. Also, a tip 200 is attached to a lower end of the tube 100 and then the ends of the first and second tubes 120 and 140 are closed.

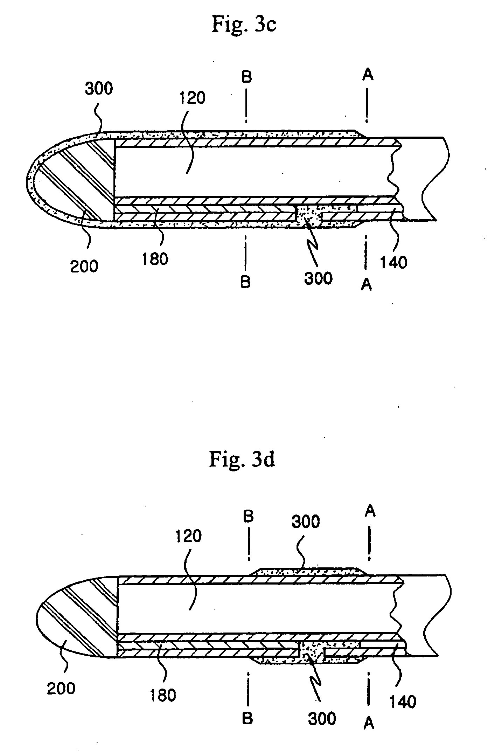

Next, the tube 100 is dipped into mold lubricant liquid (soapy water or Vaseline liquid etc.) from its end to the balloon expansion unit, especially until the line of A-A of the tube 100 and then solidified. Thereafter, the tube 100 is coated by a mold lubricant 300 until the balloon expansion unit of the outer surface of the tube. At the same time, the mold lubricant 300 is filled at the first opening 160 and a portion of the second tube 140, so that the tube is formed as shown in Figure 3c. That is, the mold lubricant is filled within the second tube from the line of A-A of the balloon expansion unit to the first opening and coated at the outer surface of the tube between the lines of A-A and B-B.

Thereafter, as shown in Figure 3d, the tube 100 is processed by surface active agent until before (the line of B-B) the balloon expansion unit, dipped into predetermined water or hot water on several times and then the mold lubricant coated except for the balloon expansion unit is removed. Thereafter, as shown in Figure 3e, many folds layers, that is two folds in the Figure 3e, are coated at the entire outer surface of the tube 100, thereby forming an overcoat layer 400.

Next, the filled and coated mold lubricant of the balloon expansion unit is removed through the second tube 140 of the tube, thereby forming a space portion 440 for expanding the balloon.

However, in case of the balloon catheter manufactured by the above processes, after dipping the tube into the mold lubricant liquid, the mold lubricant coated at a portion (between the end and the line of B-B) except for the balloon expansion opening is removed. At this time, in the dipping process of the tube in water by several times, wastewater is increased, thereby occurring environmental pollution.

Also, there is a disadvantage that when the mold lubricant did not completely removed, after forming the space portion, pushing phenomenon is occurred, so that the surrounding overcoat layer is exfoliated and swelled together with the space portion.

Also, the conventional catheter manufacturing method does not solve the problem that the diameter of the balloon portion is thicker than that of the other portion.

As an example of an another conventional art, a silicon rubber catheter is disclosed in Japanese Patent Registration No. 3015310 registered on June 21, 1995.

According to the above conventional art, a balloon is installed as one body in order to coat the surface of the catheter body. A catheter body having a discharge tube path formed by an extruding process through silicon rubber material and a drain at the outside of the tube wall is vulcanized and then mold lubricant is coated to a molding end (a portion for forming a balloon) of an insertion fixing portion. Thereafter, a wall (a balloon) is stacked at the outer surface of the tube wall of the catheter body and vulcanized again and so a tip portion is formed. At this time, the wall and the tube wall of the catheter is formed at the same surface, so that the resistance by the step portion of the wall surface is broken off and at the same time, transformation of the wall surface is prevented.

However, practically, it is impossible to manufacture the expansion tube of a continuous drain type for expanding/contracting the balloon according to the above conventional art.

Because the silicon rubber layer coated in second extruding makes the drain to be depressed. If the silicon rubber layer is coated with thinness, since the adhesion force is not sufficient, a phenomenon that a portion except for the balloon portion is exfoliated is occurred.

### SUMMARY OF THE INVENTION

To solve the above problems, it is an object of the present invention to provide a method for making a silicon balloon catheter in which since a step portion of the balloon portion is removed, pain to the patient during the surgical operation is relieved. Also, a portion except for the balloon portion is prevented from separating, at the same time productivity can be enhanced and the manufacturing cost can be reduced.

To achieve the above object, there is provided a method for making a balloon catheter using silicon rubber as defined in claim 1.

An embodiment of the present invention is disclosed in order to make a silicon rubber catheter having no a step portion economically, in which when air is injected to an expansion tube, a portion coated by mold lubricant between the first and second tubes is separated and expanded, thereby performing a function as a balloon.

### BRIEF DESCRIPTION OF THE DRAWINGS

The above objects, features and advantages of the present invention will become more apparent from the following detailed description when taken in conjunction with the accompanying drawings, in which:
Fig. 1 is a view illustrating a construction of balloon catheter in accordance with the conventional art;
Fig. 2 is a block diagram illustrating a method for making a balloon catheter in accordance with the conventional art;
Figs. 3a to 3f are longitudinal cross-sectional views illustrating processes of a balloon catheter manufacturing method in accordance with the conventional art;
Figs. 4a to 4i are longitudinal cross-sectional views illustrating processes of a balloon catheter manufacturing method in accordance with the present invention;
Fig. 5 is a cross-sectional view illustrating a construction of a balloon catheter manufactured in accordance with the present invention; and
Fig. 6 is a block diagram illustrating a method for making a balloon catheter in accordance with the present invention.

### DETAILED DESCRIPTION OF THE PREFERRED EMBODIMENT

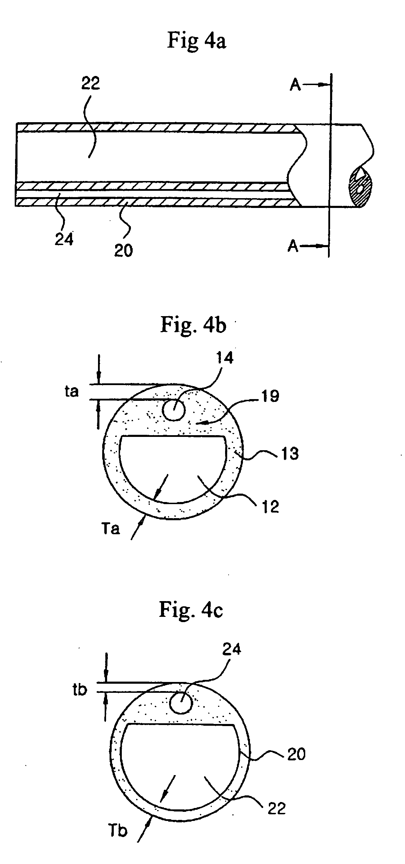

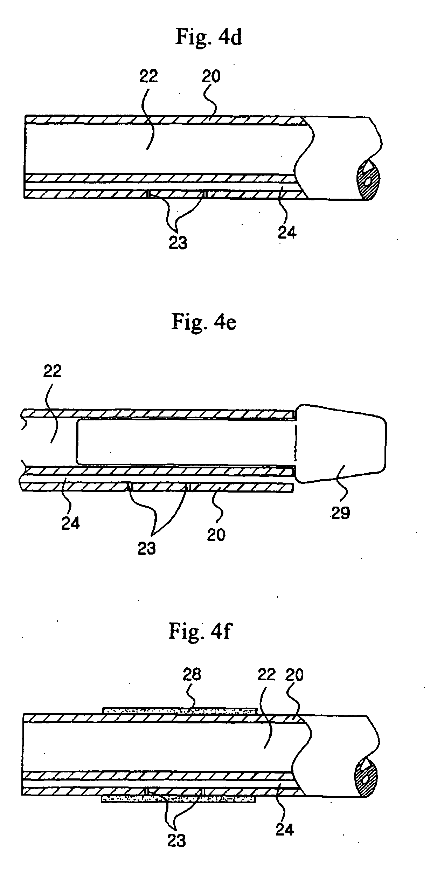

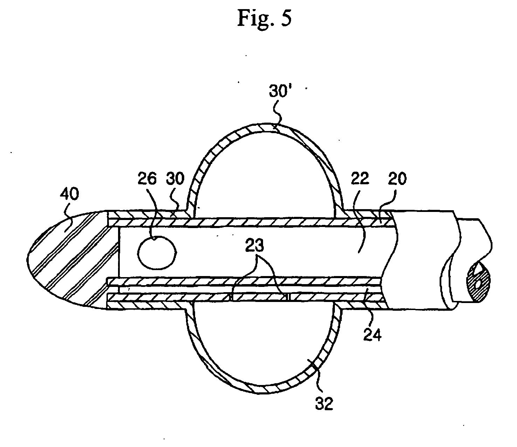

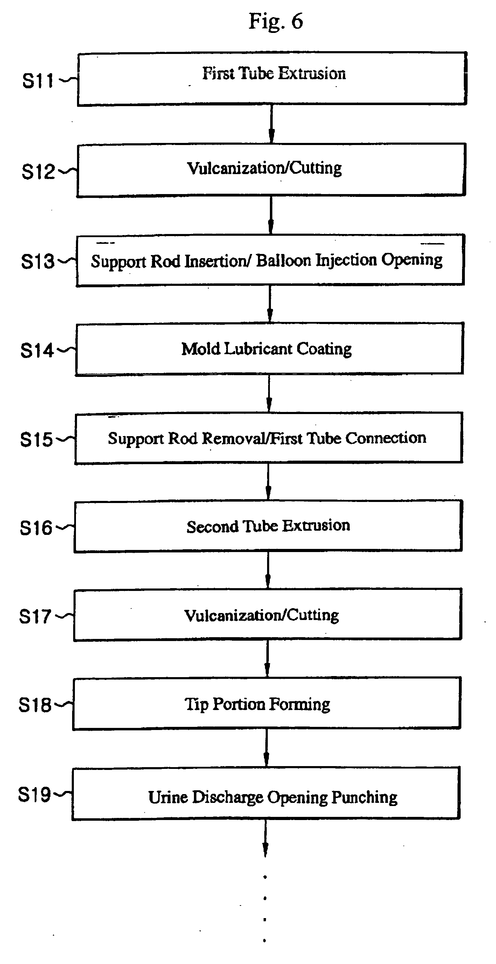

Figs. 4a and 4d to 4i are longitudinal cross-sectional views illustrating processes of a balloon catheter manufacturing method in accordance with the present invention. Fig. 5 is a cross-sectional view illustrating the construction of a balloon catheter manufactured in accordance with the present invention. Fig. 6 is a block diagram illustrating a method for making a balloon catheter in accordance with the present invention.

Firstly, as shown in Fig. 4a, in a preferred embodiment of the present invention, a first tube is formed by an extruding method. The outer diameter of the first tube is slightly smaller than the diameter of a desired balloon catheter. The first tube 20 provides with a discharge tube path 22 and an expansion tube 24 (step of S11 of Fig. 6). Thereafter, the first tube is vulcanized and as a result it has elasticity and thereafter, it is cut with a predetermined length in the step of S12.

At this time, the cross-section of the first tube 20 has the form like Fig. 4c. Fig. 4b shows a cross-section shape of the conventional art.

As shown in the above drawings, the form of the cross-section of the tube 13 of the conventional art is slightly similar to that of the first tube 20 of the present invention. However, the thickness, tb and Tb, of the first tube 20 are significantly thinner than that, ta and Ta of the conventional tube 13. This is for securing facilities in working when punching the balloon injection opening to be explained hereinafter, enhancing the rate of success, and securing a urination discharge tube path as large as it can.

Also, at the outside of the first tube 20 can form thinly as shown in Fig. 4c, considering the coated thickness again in second extruding process. In general, the thickness ta between the outside surface of the conventional tube 13 and the expansion tube 14 is 0.5mm and the thickness Ta of circumference is 0.9mm. Whereas, it is desirous that the thickness tb between the outside surface of the first tube 20 of the present invention and the expansion tube 24 is 0.3mm and the thickness Tb of circumference of the first tube 20 is 0.7mm.

Also, a horizontal type extruding machine and a vulcanizing machine are used in the conventional tube extruding S1 and vulcanization S2 processes. Whereas, it is desirous that a vertical type extruding machine and a vulcanizing machine are used in the first tube extruding S11 and vulcanization S12 processes. When the horizontal type extruding machine and the vulcanizing machine are used, some contact traces are remained to the surface of the tube, so that the traces are remained as scars at the balloon portion in next time, thereby generating eccentricity in balloon expansion or breaking the balloon.

Next, as shown in Fig. 4d, a balloon injection opening 23 is punched to the first tube 20 in step 13. At this time, a support rod 29 (referring to Fig. 4e) is inserted to the discharge tube path 22, so that the rubber tube is maintained in straight line, thereby capable of performing work easily. When forming the balloon injection opening 23, in the conventional art, an opening having a big diameter (about the diameter of 1.5mm) is punched at the center portion (15 of Fig. 1). Whereas, in an embodiment of the present invention, two openings (about the diameter of 0.5mm) are punched at an edge portion of the balloon formation portion, that is, apart from about 2~3mm from the boundary line. The two openings are as small as it can and smaller than that of the opening of the convention art.

Here, the opening of the balloon injection opening 23 is formed in small, because, if the opening is big, the balloon formation layer 30' is depressed into the opening in the second extruding process (S16 of Fig. 6) and the thickness of the balloon become to be differed. As a result, when expanding the balloon, it does not expand symmetrically and it may become a cause of breaking.

Next, as shown in Fig. 4f, mold lubricant 28 is coated to an outside portion, that is, a portion for forming the balloon, of the first tube 20 in step S14. In the mold lubricant coating process, since the mold lubricant must be coated only to the portion punched the balloon injection opening 23 in the first tube 20, turning the circumference of the outer surface of the first tube 20 by a round. Also, the coating boundary surface must be formed as a straight line, so it must take note of the work.

At this time, the used mold lubricant 28 is liquid type soap capable of purchasing easily in the street. Also, the mold lubricant 28 is used material by mixing Teflon liquid with transparent ink and it is desirous to add water or alcohol for fitting the viscosity.

As described above, the mold lubricant having the fitted viscosity is deposited in a sponge etc. and then coated only to the portion punched the balloon injection opening 23, turning the first tube by a round uniformly. After coating the mold lubricant, it is exposed to hot wind in weak with about 60~70°C in step S 14.

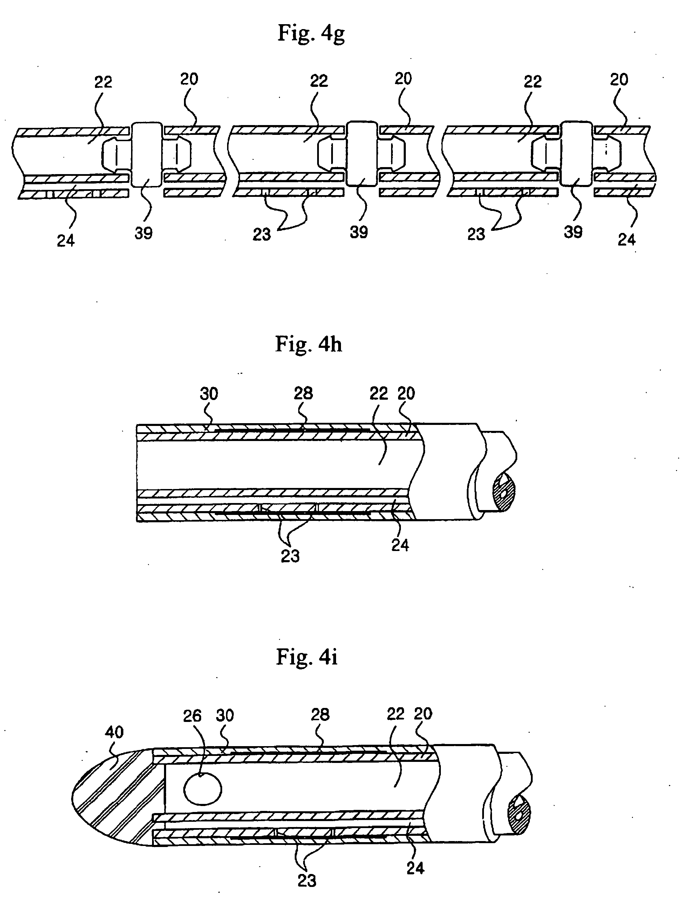

After the mold lubricant is dried sufficiently, the support rod 29 inserted to the injection tube path is removed (referring to Fig. 4f). Thereafter, the first tubes are connected with the same direction by a connecting unit 39 (referring to Fig. 4g) lengthwise in step S15, so that when extruding the second tube, a continuous working is possible.

Next, as shown in Fig. 4h, a second tube 30 is formed at the outside of the first tube 20 through the second extruding process by using a vertical type extruder in step S16 and then vulcanized by using a vulcanizing machine in step S17. At this time, the thickness of the second tube must be maintained uniformly and also it is required that the coating portion of the mold lubricant does not discolor or the characteristic of the mold lubricant does not deteriorate.

In the tube completed the vulcanizing process, the portion connected before the second extruding is cut again in step S17 and then the connection units 39 are removed, a tip 40 is formed in step S18, as shown in Fig. 4I, and then a urine discharge opening 26 is punched.

The product completed through the above processes is shown in Fig. 5, the balloon formation portion 30' is expanded symmetrically with each other when air is injected through the expansion tube 24, so that an expansion tube 32 is formed.

According to the embodiment of the present invention as described above, there is provided a method for making a silicon balloon catheter in which a first tube having its outer diameter slightly smaller than that of a desired catheter, is formed by an extruding method, mold lubricant is coated at a portion of a balloon injection opening, a thin film type second tube is formed at the coated outer surface of the first tube by a second extruding, and then the catheter is vulcanized and cut. As a result, when liquid is injected to an expansion tube, the second tube is separated from the first tube, thereby performing a function as a balloon. Also, since a step portion of the balloon portion is removed, pain to the patient during the surgical operation is relieved. A portion, except the balloon portion is prevented from separating, at the same time productivity can be enhanced and the manufacturing cost can be reduced.

While the invention has been shown and described with reference to certain preferred embodiments thereof, it will be understood by those skilled in the art that various changes in form and details may be made therein without departing from the scope of the invention as defined by the appended claims.

## Claims

1. A method for making a balloon catheter using silicon rubber comprising the steps of:
forming a first tube (20) having a discharge lumen by extruding firstly, the tube having its outer diameter slightly smaller than that of the final outer diameter of a desired catheter, then vulcanizing and cutting the first tube into lenght;
punching two balloon injection openings having small diameter in the first tube at a portion for expanding the balloon after inserting a support rod into the discharge lumen;
coating mold lubricant at a portion of the balloon injection opening;
connecting the first tubes coated with mold lubricant by using a connection unit after removing the support rod, and thereafter forming a second tube at the coated outside surface of the first tube by extruding secondly, performing a vulcanizing process and cutting again;
forming a tip at the tip portion of the first and second tubes; and
punching a urine discharge opening at the first tube.

## Patentansprüche

1. Ballonkatheter-Fertigungsverfahren mit Verwendung von Silikongummi, welches nachfolgende Schritte umfasst:
das Formen eines ersten Schlauches (20) mit einem Ausflusslumen durch ein erstes Extrudieren, wobei der Außendurchmesser des Schlauches geringfügig kleiner ist als der endgültige Außendurchmesser eines gewünschten Katheters, anschließend durch Vulkanisieren und durch Auf-Länge-Schneiden des ersten Schlauches;
das Stanzen von zwei Balloninjektionsöffnungen mit einem kleinen Durchmesser in den ersten Schlauch auf dem Ballonexpandierabschnitt nach Einführung eines Stützstabes in das Ausflusslumen;
das Auftragen eines Formgleitmittels auf dem Balloninjektionsöffnungsabschnitt;
das Verbinden der ersten mit Formgleitmittel beschichteten Schläuche durch Verwendung einer Verbindungseinheit nach Entfernung des Stützstabes, und danach das Formen eines zweiten Schlauches an der beschichteten Außenoberfläche des ersten Schlauches durch ein zweites Extrudieren, durch Ausführen eines Vulkanisierprozesses und durch wiederholtes Schneiden;
das Formen einer Spitze an der Spitzenaufnahme des ersten und zweiten Schlauches; und
das Stanzen einer Urinausflussöffnung in den ersten Schlauch.

## Revendications

1. Procédé pour fabriquer un cathéter à ballonnet en utilisant un caoutchouc de silicone comprenant les étapes de :
formation d'un premier tube (20) présentant une lumière de décharge par une première extrusion, le tube ayant son diamètre extérieur légèrement plus petit que celui du diamètre extérieur final du cathéter souhaité, puis vulcanisation et découpage du premier tube dans la longueur ;
découpe à l'emporte-pièce de deux ouvertures d'injection de ballonnet ayant un petit diamètre dans le premier tube à une partie pour la dilatation du ballonnet après insertion d'une tige de support dans la lumière de décharge ;
revêtement par du lubrifiant moulé d'une partie de l'ouverture d'injection de ballonnet ;
connexion des premiers tubes revêtus par du lubrifiant moulé par l'utilisation d'une unité de connexion après retrait de la tige de support, et ensuite formation d'un second tube sur la surface extérieure revêtue du premier tube par une seconde extrusion, réalisation d'un processus de vulcanisation et découpage à nouveau ;
formation d'un bout à la partie d'extrémité des premier et second tubes ; et découpe à l'emporte-pièce d'une ouverture de décharge d'urine sur le premier tube.
